(19) Europäisches Patentamt

European Patent Office

Office européen des brevets

(11) **EP 0 920 642 B1**

## (12) EUROPÄISCHE PATENTSCHRIFT

(45) Veröffentlichungstag und Bekanntmachung des Hinweises auf die Patenterteilung:
**15.11.2000 Patentblatt 2000/46**

(21) Anmeldenummer: 97935413.1

(22) Anmeldetag: **22.08.1997**

(51) Int Cl.⁷: **G01S 15/89**

(86) Internationale Anmeldenummer:
**PCT/CH97/00311**

(87) Internationale Veröffentlichungsnummer:
**WO 98/08112 (26.02.1998 Gazette 1998/08)**

(54) **3-D ULTRASCHALLAUFNAHMEGERÄT**

3D ULTRASOUND RECORDING DEVICE

DISPOSITIF D'ENREGISTREMENT D'IMAGES ULTRASONORES TRIDIMENSIONNELLES

(84) Benannte Vertragsstaaten:
**AT CH DE DK ES FI FR GB IE IT LI NL PT SE**

(30) Priorität: **22.08.1996 CH 206296**

(43) Veröffentlichungstag der Anmeldung:
**09.06.1999 Patentblatt 1999/23**

(73) Patentinhaber: **SYNTHES AG Chur**
**7002 Chur (CH)**

(72) Erfinder:
• **EMMENEGGER, Niklaus**
**CH-8032 Zürich (CH)**

• **ENGFER, Olaf**
**CH-8050 Zürich (CH)**

(74) Vertreter: **Lusuardi, Werther Giovanni, Dr. et al**
**Dr. Lusuardi AG,**
**Kreuzbühlstrasse 8**
**8008 Zürich (CH)**

(56) Entgegenhaltungen:
**WO-A-94/23647        US-A- 4 396 945**
**US-A- 4 896 673        US-A- 5 197 476**
**US-A- 5 198 877**

EP 0 920 642 B1

**Beschreibung**

Die Erfindung bezieht sich auf ein Ultraschallaufnahmegerät gemäss Patentanspruch 1, eine Vorrichtung zur Aufnahme von dreidimensionalen Ultraschallbildern gemäss dem abhängigen Patentanspruch 19 sowie auf ein Verfahren zur Aufnahme von dreidimensionalen Ultraschallbildern gemäss dem Patentanspruch 34.

Ein System zur Erfassung der Position eines Messfühlers innerhalb eines Objektes und zur Anzeige von vorher aufgenommenen, zur erfassten Position entsprechenden Bildern des Objektes ist aus der Patentschrift US 5,383,454 BUCHHOLZ bekannt. Dieses System ermöglicht ebenfalls, die Spitze eines Messfühlers in einem Objekt an einen bestimmten Ort hinzuführen, wobei die Lage des Messfühlers nur an einem Bildschirm, welcher gleichzeitig auch ein vorher aufgenommenes Bild dieses Teils des Objektes wiedergibt, beobachtet werden kann. Die Position des Messfühlers wird bei dieser bekannten Erfindung durch einen handelsüblichen dreidimensionalen Schalldigitizer ermittelt.

Ein weiteres Verfahren und eine Vorrichtung zur Erfassung von diagnostisch verwertbaren, dreidimensionalen Ultraschallbilddatensätzen ist beispielsweise aus der Patentschrift EP 0 736 284 A2 POLZ bekannt. Die bekannte Erfindung beinhaltet eine Vorrichtung, mit der bei freier manueller Führung des Vorrichtung, mit der bei freier manueller Führung des Ultraschallkopfes eine tomographische Erfassung eines gesamten, zu untersuchenden Volumens oder dreidimensionalen Raumes durch einen dreidimensionalen Datensatz möglich ist. Die Lage und Orientierung des Ultraschallkopfes wird durch ein weiteres elektromagnetisch arbeitendes Sensorsystem erfasst. Am Ultraschallkopf, der von der untersuchenden Person manuell frei geführt wird, ist vorzugsweise ein Halter vorgesehen, an welchem sich auch der Empfänger dieses elektromagnetisch arbeitenden Sensorsystems befindet. Dieses Sensorsystem liefert aufgrund der von einem Sender abgestrahlten Magnetfelder, die von dem Empfänger, bzw. den dortigen Spulen erfasst werden, an seinem Ausgang Sensordaten (Positions- und Rotationsdaten), mit denen die Position und Orientierung des Ultraschallkopfes mit dem Empfänger im Raum genau definiert ist, und zwar durch Translationsdaten in X-, Y- und Z-Achse sowie durch Rotationsdaten um diese Achsen.

Soll bei der Lage- und Orientierungsbestimmung mittels Magnetfeldvermessung eine genügende Genauigkeit erreicht werden, so müssen äussere Parameter wie:

- Störfelder, wie sie beispielsweise durch Bildschirme, Computer oder Elektromotoren hervorgerufen werden;

- Störungen, die durch hochpermeable Stoffe im Magnetfeld, beispielsweise durch Metalle, die im Messbereich bewegt werden, entstehen; oder

- durch die Wechselstromversorgung hervorgerufene elektromagnetische Störfelder

sehr genau bekannt sein. Die Erfassung dieser Effekte und/oder deren Minimierung durch konstruktive Massnahmen, beispielsweise durch Abschirmungen oder fortlaufende Kalibration, bedarf grosser Aufwendungen. Der Nachteil dieser bekannten Vorrichtung liegt daher darin, dass die geforderte Genauigkeit bei der Bestimmung der Lage und der Orientierung schwer zu erreichen ist.

Eine weitere Vorrichtung zur Aufnahme von Ultraschallbildern mit Hilfe eines manuell frei bewegbaren Ultraschallaufnahmekopfes ist aus der US 5,197,476 NOWACKI bekannt. Diese bekannte Vorrichtung dient zur Ortung eines Zielobjektes innerhalb eines menschlichen Körpers. Die Vorrichtung umfasst einen dreidimensionalen Rahmen, welcher mit einer Anzahl Infrarot-Leuchtdioden versehen ist und auf einem Tisch positioniert wird, ein Paar Infrarotkameras zur Aufnahme der von den Infrarot-Leuchtdioden emittierten Strahlung, einen Computer und eine Ultraschallsonde, welche ebenfalls mit Infrarot-Leuchtdioden versehen ist. Vor dem Einsatz der Ultraschallsonde wird der Rahmen auf dem Tisch aufgebaut und mittels der Kameras die Position der Infrarot-Leuchtdioden vermessen und im Computer gespeichert. Anschliessend wird der menschliche Körper innerhalb des Referenzvolumens oder unmittelbar daran anschliessend plaziert. Die manuell frei bewegbare Ultraschallsonde wird innerhalb des durch den dreidimensionalen Rahmen definierten Referenzvolumens bewegt, so dass sich die Ultraschallsonde im Messvolumen der Kameras befindet. Die Position der an der Ultraschallsonde angebrachten Infrarot-Leuchtdioden wird durch den Computer mit den Anfangspositionen der Infrarot-Leuchtdioden auf dem dreidimensionalen Rahmen verglichen, wodurch sich die Position der Ultraschallsonde sehr genau bestimmen lässt und die Position des Zielobjektes auf einem Computerbildschirm darstellbar ist. Nachteil dieser bekannten Erfindung ist, dass die Ultraschallaufnahmen nur innerhalb des vorgängig mittels des dreidimensionalen Rahmens definierten Referenzvolumens aufgenommen werden können.

Eine Methode zur Bestimmung der Lage und Orientierung einer Plattform im Raum wird in der US 4,396,945 DI MATTEO offenbart. Die Mittel zur eindeutigen Identifikation der drei an der Plattform angebrachten Lichtquellen umfassen drei Lichtmodulatoren, welche zwischen der Lichtquelle und den fiberoptischen Verbindungen angebracht sind. Jeder der drei Lichtmodulatoren wird durch einen Codegenerator mit einem eindeutigen Code versorgt, wodurch eine Ein-Aus Modulation jeder Lichtquelle erzeugt wird. In einer anderen Ausführungsform dieser bekannten Methode wird die eindeutige Identifikation jeder Lichtquelle dadurch erreicht, dass jede Lichtquelle mit einem Farbreflektor zur Reflektion einer bestimmten von den anderen Lichtquellen verschiedenen Farbe versehen ist. Der Nachteil dieser bekannten Erfindung liegt darin, dass die auf dem bewegten Objekt angebrachten drei Lichtquellen mit einer Ein-Aus Modulation oder wenn es sich im Reflektoren handelt, diese mit einer Farbkennung versehen sein müssen.

Hier will die Erfindung Abhilfe schaffen. Der Erfindung liegt die Aufgabe zugrunde, die Aufnahme von dreidimensionalen Ultraschallbildern mittels einem manuell frei bewegbaren Ultraschallaufnahmekopf , einem Ultraschallaufnahmegerät und einer Positionserfassungseinrichtung zu ermöglichen, wobei die Positionserfassungseinrichtung es erlaubt, die Position und Orientierung des Ultraschallaufnahmekopfes und damit die Lage und Orientierung der Ultraschallschnittbilder im Raum zu einer beliebigen Basis durch Längenmessung zu bestimmen.

Gegenüber der aus der US 5,197,476 NOWACKI bekannten Erfindung, wo zur Aufnahme der Ultraschallbilder vorgängig mittels eines dreidimensionalen Rahmens ein Referenzvolumen definiert werden muss, ermöglicht die vorliegende Erfindung, dass die Basis, welche zur Lagebestimmung der Ultraschallsonde dient, durch Empfänger gebildet wird, also auch durch die zur Positionserfassung der Ultraschallsonde dienenden Kameras gebildet werden kann.

Die durch die Erfindung erreichten Vorteile sind im wesentlichen darin zu sehen, dass dank der erfindungsgemässen Vorrichtung die folgenden vereinfachenden Bedingungen zur Handhabung erreicht werden:

- im Bereich der Auflösungsgenauigkeit keine Beeinflussung des Systems durch äussere Parameter;

- einfaches Handling des Systems, auch wenn einmal die Lageerfassung unterbrochen werden sollte, beispielsweise durch ein Objekt, das zwischen Aufnahmegerät und Ultraschallkopf geriet, kann bei freier Sicht sofort weitergemessen werden; und

- keine Beeinflussung der Trackinggenauigkeit durch äussere elektromagnetische Felder von Bildschirmen und/oder elektrischen Geräten.

Die Erfindung löst die gestellte Aufgabe mit einem Ultraschallaufnahmegerät, welches die Merkmale des Anspruches 1 aufweist, einer Vorrichtung zur Aufnahme von dreidimensionalen Ultraschallbildern, welche die Merkmale des Anspruchs 19 aufweist, sowie einem Verfahren zur Aufnahme von dreidimensionalen Ultraschallbildern, welches die Merkmale des Anspruchs 34 aufweist.

Eine Ausführungsform der erfindungsgemässen Vorrichtung besteht darin, dass die zur Lage- und Positionserfassung am Ultraschallkopf angebrachten, elektromagnetische Wellen abgebenden Mittel optische Leuchtquellen sind.

Eine weitere Ausführungsform der erfindungsgemässen Vorrichtung besteht darin, dass die zur Lage- und Positionserfassung am Ultraschallkopf angebrachten, elektromagnetische Wellen abgebenden Mittel oder Infrared Light Emitting Dioden (IRED) sind.

Eine andere Ausführungsform der erfindungsgemässen Vorrichtung besteht darin, dass die zur Lage- und Positionserfassung am Ultraschallkopf angebrachten, elektromagnetische Wellen abgebenden Mittel Reflektoren oder Fluoreszenzreflektoren sind.

Wieder eine weitere Ausführungsform der erfindungsgemässen Vorrichtung besteht darin, dass die zur Lage- und Positionserfassung am Ultraschallkopf angebrachten, elektromagnetischen Wellen abgebenden Mittel durch eine Lichtquelle gespiesene faseroptische Lichtleiter sind.

Wiederum eine andere Ausführungsform der erfindungsgemässen Vorrichtung besteht darin, dass die im Raum diese elektromagnetischen Wellen detektierenden Sensorsysteme raumfeste eindimensionale Kameras sind und somit durch eine Auswerteeinheit die Position und Orientierung des Ultraschallkopfes und damit die Lage und Orientierung der Ultraschallschnittbilder im Raum ermittelt werden kann.

Eine weitere Ausführungsform der erfindungsgemässen Vorrichtung besteht darin, dass die im Raum diese elektromagnetischen Wellen detektierenden Sensorsysteme Kameras sind, die nicht raumfest angeordnet sind und die Lage der Kameras durch Aufnahme und Auswertung eines raumfesten Passpunktfeldes erfolgt und somit durch die Auswerteeinheit die Position und Orientierung des Ultraschallkopfes und damit die Lage und Orientierung der Ultraschallschnittbilder im Raum ermittelt werden kann. Durch die Aufnahme und Auswertung des raumfesten Passpunktfeldes sind dann real-time Messungen sogar unter instabilen Umgebungsbedingungen möglich. Immer dann wenn die Kameras ein Bild aufnehmen, werden mit Hilfe des Passpunktfeldes die aktuellen Kamerapositionen neu berechnet. Lageänderungen der Kameras lassen sich somit vollständig kompensieren.

Eine andere Ausführungsform der erfindungsgemässen Vorrichtung besteht darin, dass die im Raum diese elektromagnetischen Wellen detektierenden Sensorsysteme raumfest angeordnet sind und somit die Lage und Orientierung eines nicht raumfesten Passpunktfeldes, das beispielsweise am Patienten angebracht ist, ermittelt werden kann.

Wiederum eine andere Ausführungsform der erfindungsgemässen Vorrichtung besteht darin, dass die mindestens zwei im Raum diese elektromagnetischen Wellen detektierenden Sensoren raumfeste Kameras sind und somit durch eine Auswerteeinheit videogrammetrisch die Position und Orientierung des Ultraschallkopfes und damit die Lage und Orientierung der Ultraschallschnittbilder im Raum ermittelt werden kann.

Eine weitere Ausführungsform der erfindungsgemässen Vorrichtung besteht darin, dass die mindestens zwei im Raum diese elektromagnetischen Wellen detektierenden Sensoren Kameras sind, die nicht raumfest angeordnet sind und die Lage der Kameras durch Aufnahme und Auswertung eines raumfesten Passpunktfeldes erfolgt und somit durch die Auswerteeinheit videogrammetrisch die Position und Orientierung des Ultraschallkopfes damit die Lage und Orientierung der Ultraschallschnittbilder im Raum ermittelt werden kann. Durch die Aufnahme und Auswertung des raumfesten Passpunktfeldes sind dann real-time Messungen sogar unter instabilen Umgebungsbedingungen möglich. Immer dann wenn die Kameras ein Bild aufnehmen, werden mit Hilfe des Passpunktfeldes die aktuellen Kamerapositionen neu berechnet. Lageänderungen der Kameras lassen sich somit vollständig kompensieren.

Wiederum eine weitere Ausführungsform der erfindungsgemässen Vorrichtung besteht darin, dass der manuell frei bewegbare Ultraschallkopf, das Ultraschallaufnahmegerät, das Bildverarbeitungssystem, und die Positionserfassungseinrichtung mit einem Computer Assisted Surgery System (CAS) verbunden sind.

Eine Ausführungsform des erfindungsgemässen Verfahrens besteht darin, dass die zur Lage- und Positionserfassung am Ultraschallkopf angebrachten, elektromagnetische Wellen abgebenden Mittel optische Leuchtquellen sind.

Eine weitere Ausführungsform des erfindungsgemässen Verfahrens besteht darin, dass die zur Lage- und Positionserfassung am Ultraschallkopf angebrachten, elektromagnetische Wellen abgebenden Mittel oder Infrared Light Emitting Dioden (IRED) sind.

Eine andere Ausführungsform des erfindungsgemässen Verfahrens besteht darin, dass die zur Lage- und Positionserfassung am Ultraschallkopf angebrachten, elektromagnetische Wellen abgebenden Mittel Reflektoren oder Fluoreszenzreflektoren sind.

Wieder eine weitere Ausführungsform des erfindungsgemässen Verfahrens besteht darin, dass die zur Lage- und Positionserfassung am Ultraschallkopf angebrachten, elektromagnetischen Wellen abgebenden Mittel durch eine Lichtquelle gespiesene faseroptische Lichtleiter sind.

Wiederum eine andere Ausführungsform des erfindungsgemässen Verfahrens besteht darin, dass die im Raum diese elektromagnetischen Wellen detektierenden Sensorsysteme raumfeste eindimensionale Kameras sind und somit durch eine Auswerteeinheit die Position und Orientierung des Ultraschallkopfes und damit die Lage und Orientierung der Ultraschallschnittbilder im Raum ermittelt werden kann.

Eine weitere Ausführungsform des erfindungsgemässen Verfahrens besteht darin, dass die im Raum diese elektromagnetischen Wellen detektierenden Sensorsysteme Kameras sind, die nicht raumfest angeordnet sind und die Lage der Kameras durch Aufnahme und Auswertung eines raumfesten Passpunktfeldes erfolgt und somit durch die Auswerteeinheit die Position und Orientierung des Ultraschallkopfes und damit die Lage und Orientierung der Ultraschallschnittbilder im Raum ermittelt werden kann. Durch die Aufnahme und Auswertung des raumfesten Passpunktfeldes sind dann real-time Messungen sogar unter instabilen Umgebungsbedingungen möglich. Immer dann wenn die Kameras ein Bild aufnehmen, werden mit Hilfe des Passpunktfeldes die aktuellen Kamerapositionen neu berechnet. Lageänderungen der Kameras lassen sich somit vollständig kompensieren.

Eine andere Ausführungsform des erfindungsgemässen Verfahrens besteht darin, dass die im Raum diese elektromagnetischen Wellen detektierenden Sensorsysteme raumfest angeordnet sind und somit die Lage und Orientierung eines nicht raumfesten Passpunktfeldes, das beispielsweise am Patienten angebracht ist, ermittelt werden kann.

Wiederum eine andere Ausführungsform des erfindungsgemässen Verfahrens besteht darin, dass die mindestens zwei im Raum diese elektromagnetischen Wellen detektierenden Sensoren raumfeste Kameras sind und somit durch eine Auswerteeinheit videogrammetrisch die Position und Orientierung des Ultraschallkopfes und damit die Lage und Orientierung der Ultraschallschnittbilder im Raum ermittelt werden kann.

Eine weitere Ausführungsform des erfindungsgemässen Verfahrens besteht darin, dass die mindestens zwei im Raum diese elektromagnetischen Wellen detektierenden Sensoren Kameras sind, die nicht raumfest angeordnet sind und die Lage der Kameras durch Aufnahme und Auswertung eines raumfesten Passpunktfeldes erfolgt und somit durch die Auswerteeinheit videogrammetrisch die Position und Orientierung des Ultraschallkopfes damit die Lage und Orientierung der Ultraschallschnittbilder im Raum ermittelt werden kann. Durch die Aufnahme und Auswertung des raumfesten Passpunktfeldes sind dann real-time Messungen sogar unter instabilen Umgebungsbedingungen möglich. Immer dann wenn die Kameras ein Bild aufnehmen, werden mit Hilfe des Passpunktfeldes die aktuellen Kamerapositionen neu berechnet. Lageänderungen der Kameras lassen sich somit vollständig kompensieren.

Wiederum eine weitere Ausführungsform des erfindungsgemässen Verfahrens besteht darin, dass der manuell frei bewegbare Ultraschallkopf, das Ultraschallaufnahmegerät, das Bildverarbeitungssystem, und die Positionserfassungseinrichtung mit einem Computer Assisted Surgery System (CAS) verbunden sind.

Die in der Erfindung verwendeten Grundlagen zur optischen, grammetrischen Positionserfassung können unter anderen im folgenden Lehrbuch nachgeschlagen werden:

Jordan/Eggert/Kneissl

Handbuch der Vermessungskunde
10. völlig neu bearb. Ausgabe
Band IIIa/3
Photogrammetrie
J.B. Metzlersche Verlagsbuchhandlung, Stuttgart, 1972 (insbesondere §§ 144, 145, 146, 147)

Die in den Patentansprüchen auftretenden Begriffe Interferenz- und Längenmessung beziehen sich also nicht nur auf Interferenzmessungen wie sie z.B. bei Laserdistanzmessungen auftreten, sondern insbesondere auch auf die Interferenzeffekte, mittels derer Abbildungen durch optische Systeme (z.B.zentralperspektivische Abbildungen) in einer Bildebene oder Bildgeraden entstehen.

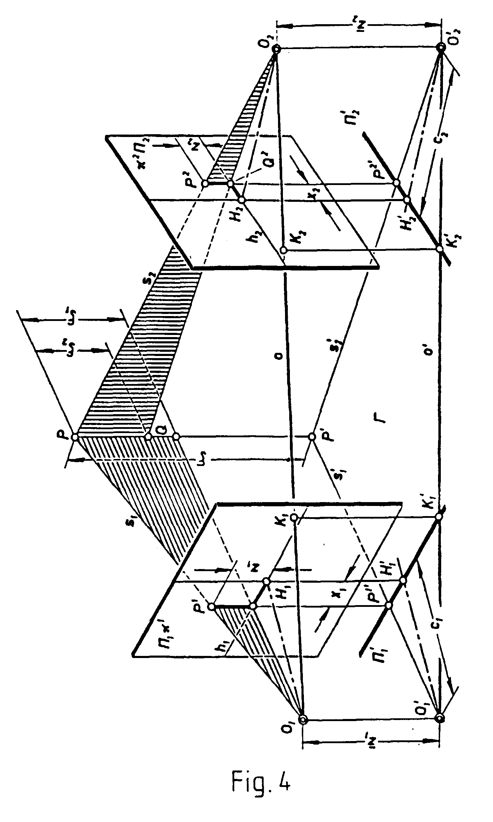

Des weiteren sind als Längenmessungen sowohl Längenmessungen auf einer Bildebene (bzw. Bildgeraden) zu verstehen (z.B. auf einem CCD-Chip), d.h. z.B. die Längenmessung der Strecke $z_1$, $z_2$ in Fig. 4 (§146.2, Fig. 5, in Handbuch der Vermessungskunde), als auch absolute Längenvermessung des zu bestimmenden Objektes, wie sie z.B. in Laufzeitbestimmungsmethoden anzutreffen sind (wie z.B. bei einem GPS-System).

Anstelle der in Fig. 4 beschriebenen Methode mittels zweier Projektionsebenen kann auch eine ebenfalls auf dem Strahlensatz beruhende Vermessungsmethode mittels mindestens 3 nicht kolinearen 1-dimensionalen CCD-Chips angewandt werden. Ein Produkt ist unter dem Namen Optotrak™ erhältlich.

Sind die Kameras der erfindungsgemässen Vorrichtung mit CCD-Chips ausgerüstet, so können vier nicht komplanare Punkte auf den CCD-Chips der Kameras die minimale Empfängerbasis bilden. Dann gilt, dass die Vermessung (Längenmessung) beispielsweise videogrammetrisch oder mittels Strahlensatz erfolgt, d.h. die Bilder auf den CCD-Chips werden vermessen. Dabei ist die Basis wegen der Möglichkeit einer Relativmessung auf den CCD-Chips beliebig wählbar, was auch eine beliebige Positionierbarkeit der Kameras ermöglicht. Daraus folgt, dass die gegenseitige Lage der Basispunkte auf den CCD-Chips bestimmt werden muss, was ohne Referenzvolumen, beispielsweise durch eine Abstandmessung von Kamera zu Kamera möglich ist.

Die Erfindung und Weiterbildungen der Erfindung werden im folgenden anhand der teilweise schematischen Darstellungen mehrerer Ausführungsbeispiele noch näher erläutert.

Es zeigen:

Fig. 1 eine schematische Darstellung einer Ausführungsform der erfindungsgemässen Vorrichtung;

Fig. 2 eine schematische Darstellung einer weiteren Ausführungsform der erfindungsgemässen Vorrichtung;

Fig. 3 eine schematische Darstellung einer weiteren Ausführungsform der erfindungsgemässen Vorrichtung; und

Fig. 4 eine schematische Darstellung zur Erläuterung des grammetrischen Verfahrens.

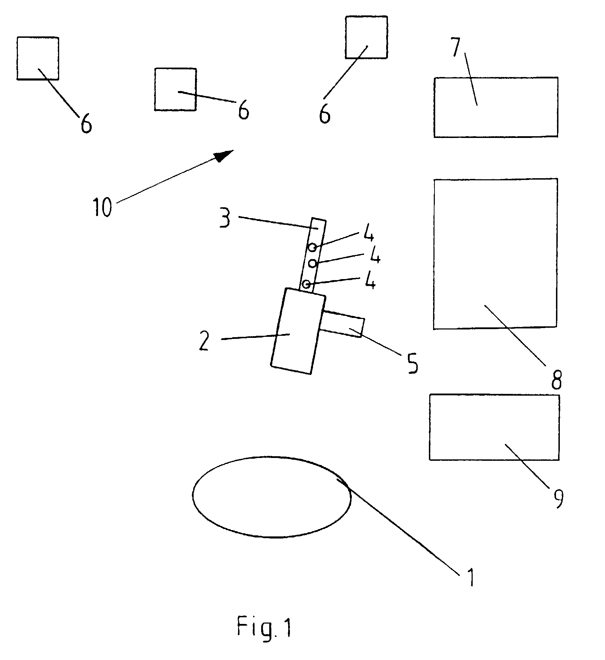

Die in Fig. 1 dargestellte Ausführungsform der erfindungsgemässen Vorrichtung zeigt einen manuell frei bewegbaren Ultraschallkopf 2, ein Ultraschallaufnahmegerät 9, ein Bildverarbeitungssystem 8 und eine Positionserfassungseinrichtung 10 zur Aufnahme von dreidimensionalen Ultraschallbildern des Körpers 1. Die Positionserfassungseinrichtung 10 gestattet die Position und Orientierung des Ultraschallkopfes 2 und damit die Lage und Orientierung der Ultraschallschnittbilder im Raum zu bestimmen. Am Ultraschallkopf 2 sind Sender 4 angebracht, die elektromagnetische Wellen emittieren. Zur Aufnahme der von den Sendern 4 emittierten elektromagnetischen Wellen stehen raumfeste Kameras 6, beispielsweise digitale Kameras, zur Verfügung. Die von den Sendern 4 emittierten elektromagnetischen Wellen werden von den Kameras 6 erfasst und die Sender 4 am Ultraschallkopf 2 abgebildet. In der Auswerteeinheit 7 werden dann aus den Bildern die Position und die Orientierung des Ultraschallkopfes 2 errechnet. Der Ultraschallkopf 2 kann mit Hilfe eines Handgriffes 5 frei bewegt werden, was eine tomographische Erfassung eines gesamten dreidimensionalen Körpers 1 durch einen im Bildverabeitungssystem bestimmten dreidimensionalen Datensatzes ermöglicht.

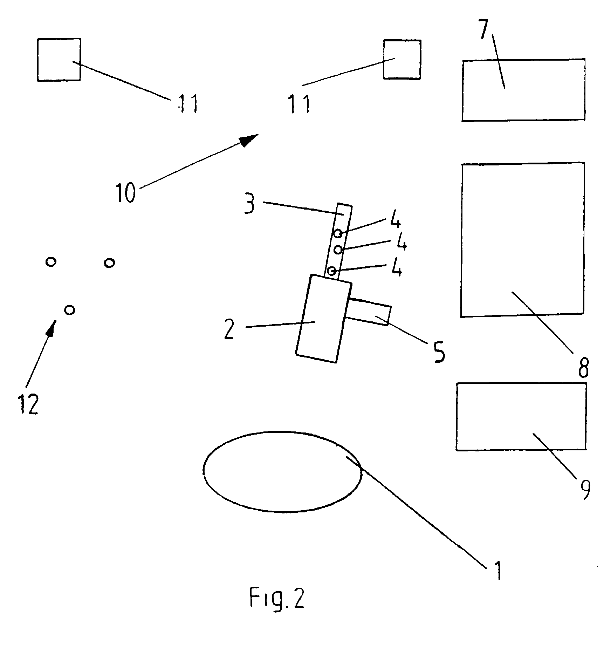

In Fig. 2 ist eine Ausführungsform der erfindungsgemässen Vorrichtung dargestellt, welche einen manuell frei bewegbaren Ultraschallkopf 2, ein Ultraschallaufnahmegerät 9, ein Bildverarbeitungssystem 8, eine Positionserfassungseinrichtung 10 und ein aus Light Emitting Dioden (LED) bestehendes Passpunktfeld 12 zur Aufnahme von dreidimensionalen Ultraschallbildern des Körpers 1 umfasst. Die Positionserfassungseinrichtung 10 gestattet die Position und Orientierung des Ultraschallkopfes 2 und damit die Lage und Orientierung der Ultraschallschnittbilder im Raum zu bestimmen. Am Ultraschallkopf 2 sind Sender 4 angebracht, die elektromagnetische Wellen emittieren. Zur Aufnahme der von den Sendern 4 am Ultraschallkopf 2 emittierten elektromagnetischen Wellen stehen zwei Kameras 6, beispielsweise digitale Kameras, zur Verfügung. Die Kameras 6 sind bei dieser Ausführungsform der erfindungsgemässen Vorrichtung nicht raumfest angeordnet und die Lage der Kameras 11 wird durch Aufnahme und Auswertung der Bilder eines raumfesten Passpunktfeldes 12 ermittelt. Die von den Sendern 4 emittierten elektromagnetischen Wellen werden von den beiden Kameras 6 erfasst und die Sender 4 auf je einer Bildebene abgebildet. In der Auswerteeinheit 7 werden dann aus den perspektivischen Verzerrungen der beiden Bilder die Position und die Orientierung des Ultraschallkopfes 2 errechnet. Der Ultraschallkopf 2 kann mit Hilfe eines Handgriffes 5 frei bewegt werden, was eine tomographische Erfassung eines gesamten dreidimensionalen Körpers 1 durch einen im Bildverabeitungssystem bestimmten dreidimensionalen Datensatzes ermöglicht.

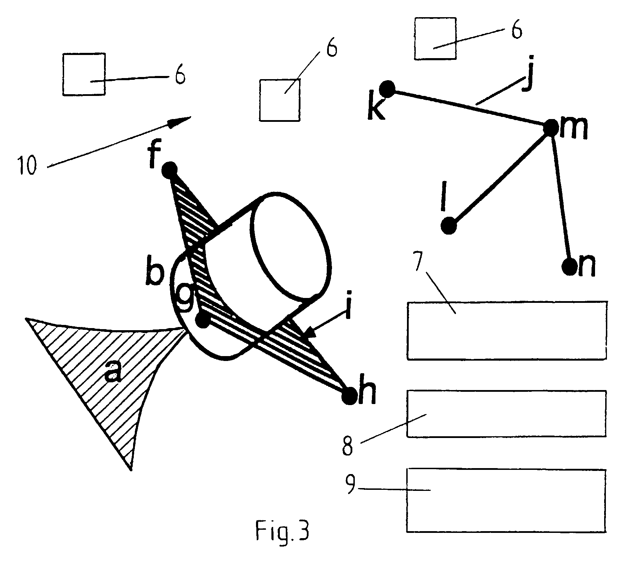

Die in Fig. 3 dargestellte Ausführungsform der erfindungsgemässen Vorrichtung zeigt einen manuell frei bewegbaren Schallkopf b, ein Ultraschallaufnahmegerät 9, ein Bildverarbeitungssystem 8 und eine Positionserfassungseinrichtung 10 zur Aufnahme von Ultraschallbildern a. Die Positionserfassungseinrichtung 10 gestattet die Position und Orientierung des Schallkopfes b und damit die Lage und Orientierung der Ultraschallschnittbilder a im Raum zu bestimmen. Mit dem Schallkopf verbunden sind fest gewählte Sender f;g;h angebracht, die elektromagnetische Wellen emittieren. Zur Aufnahme der von den Sendern f;g;h emittierten elektromagnetischen Wellen stehen raumfeste Kameras 6, beispielsweise digitale Kameras, zur Verfügung. Die von den Sendern f;g;h emittierten elektromagnetischen Wellen werden von den Kameras 6 erfasst. In der Auswerteeinheit 7 werden dann aus den erfassten Bilder die Position und die Orientierung des Schallkopfes b errechnet. Der Schallkopf b kann mit Hilfe eines Handgriffes frei bewegt werden, was eine tomographische Erfassung eines gesamten dreidimensionalen Körpers durch einen im Bildverarbeitungssystem bestimmten dreidimensionalen Datensatzes ermöglicht.

Mit Hilfe von Fig. 4 soll noch die grammetrische Methode am speziellen Beispiel der "Rekonstruktion (der Koordinaten) aus zwei Perspektiven mit bekannter gegenseitiger Lage der Bildebenen und bekannten inneren Orientierung" erklärt werden. Aus: Jordan/Eggert/Kneissl, Handbuch der Vermessungskunde, 1972, S. 2271:

146.2 Rekonstruktion aus zwei Perspektiven mit bekannter gegenseitiger Lage der Bildebenen und bekannten inneren Orientierungen:

Mit den inneren Orientierungen kennt man die Sehstrahlbündel $[O_1]$, $[O_2]$ sowie ihre Lage zu den Bildebenen. Bekannte gegenseitige Lage der Bildebenen bedeutet daher bekannte gegenseitige Lage der Sehstrahlbündel. Aus der bekannten Raumlage von $\pi_1$, $\pi_2$, $O_1$, $O_2$ gewinnt man die Kernachse o, die Gerade s=$(\pi_1 \pi_2)$, die Kernpunkte $K_1$, $K_2$ und die perspektive Zuordnung der Kernstrahlbüschel bezüglich s. Für jedes beliebige an entsprechende Kernstrahlen gebundene Bildpaar $P^1$, $P^2$ ist dann garantiert, dass sich die Sehstrahlen $s_1=[O_1P^1]$ und $s_2=[O_2P^2]$ in einem Raumpunkt P schneiden. Man kennt damit die Lage von P im System der Sehstrahlbündel. Die Bestimmung der Lage von P in einem gegebenen räumlichen Bezugssystem S erfordert die Kenntnis der Lage von 1, 2 in S. Falls letztere nicht unmittelbar gegeben ist, ist sie nach §145.3 zu bestimmen. Als Beispiel einer nicht instrumentell durchgeführten Rekonstruktion behandeln wir nachfolgend die sog. Messtischphotogrammetrie.

a) Messtischphotogrammetrie (Fig. 4) <(a) einfachste Darstellung, wobei die Bildebenen 1, 2 durch CCD-Chips zu belegen sind>

$\Gamma$ sei eine horizontale Ebene (Grundrissebene). Die Bildebenen $\pi_1$, $\pi_2$ seien vertikal, also die Hauptsehstrahlen $[O_1H_1]$, $[O_2,H_2]$ horizontal. $h_1$, $h_2$ seien die Bildhorizonte in $\pi_1$, $\pi_2$. $x_1$, $z_1$ bzw. $x_2$, $z_2$ seien Bildkoordinaten in 1 bzw. 2. Ursprung des jeweiligen Bildkoordinatensystems sei der Hauptpunkt, die x-Achse liege im Horizont. $\bar{z}_1$, $\bar{z}_2$ seien die Höhen der Zentren $O_1$, $O_2$ über $\Gamma$.

Aus den Koordinaten $x_1$, $x_2$ gegebener Bildpunkte $P^1$, $P^2$ kann man in die bekannte Grundrisse $\pi_1'$, $\pi_2'$ auch die Grundrisse $P^{1'}$, $P^{2'}$ eintragen und findet den Grundriss P' des zu rekonstruierenden Raumpunktes P als Schnitt der Sehstrahlgrundrisse $s_1'=[O_1'P_1']$ und $s_2'=[O_2'P_2']$ (Vorwärtsabschnitt). Während man dabei die Standlinie $O_1'O_2'$ im Kartenmassstab aufträgt, wird man die Bildweiten und x-Koordinaten mit einem geeigneten Faktor so durchmultiplizieren, dass sich $s_1'$, $s_2'$ genügend genau zeichnen lassen.

Aus den in Fig. 4 ersichtlichen ähnlichen Dreiecken $O_2PQ$ und $O_2P^2Q^2$ findet man die Höhe $\zeta_2$ von P über der Ebene $[O_2h_2]$ mit

$$\zeta_2 = \frac{z_2 \cdot O_2' P'}{O_2' P_2'}$$

Daraus ergibt sich die Höhe $\zeta$ von P über $\Gamma$ mit $\zeta = \bar{z}_2 + \zeta_2$. Berechnet man analog $\zeta = \bar{z}_1 + \zeta_1$, so kann man die Fehler ausgleichen.

Wie Fig. 4 zeigt, findet man die Grundrisse $K_1'$, $K_2'$ der Kernpunkte $K_1$, $K_2$ als Schnittpunkte der Standlinie o'=$[O_1'O_2']$ mit $\pi_1'$, $\pi_2'$ und ihre Höhen über $\Gamma$ - also ihre Lage in $\pi_1$, $\pi_2$ - durch Umklappung des Trapezes $o_1'o_2'o_2o_1$ unter Mitnahme der lotrechten Trägergeraden für $K_1$ und $K_2$. Die Kernstrahlen werden gebraucht, um in den Bildern von Objektkurven entsprechende Punkte identifizieren zu können.

Lägen die Bildebenen $\pi^{1*}$, $\pi^{2*}$ allgemein im Raum, so könnte man leicht auf den eben behandelten Fall lotrechter Bildebenen $\pi_1$, $\pi_2$ zurückkommen. Man braucht dazu nur $\pi^{1*}$ aus $O_1$ auf $\pi_1$ und $\pi^{2*}$ aus $O_2$ auf $\pi_2$ umzuprojizieren. Ohne solches Umprojizieren ergäbe sich als Gesamtheit der Punkte P' nach Fig. 4 der Normalriss des photographierten Objektes auf eine zu $\pi^{1*}$ und $\pi^{2*}$ normale Ebene, und $\zeta$ wäre der Abstand des Punktes P von dieser Ebene.

**Patentansprüche**

1. Ultraschallaufnahmegerät zur Aufnahme von Ultraschallbildern mittels elektronischer Datenverarbeitung, wobei

   A) die Lage der Ultraschallschnittbilder (a) zu einer beliebigen Basis im Raum eindeutig bestimmt ist;

B) die Lage und Position der Ultraschallschnittbilder (a) durch die Lage und Position des Schallkopfes (b) bestimmt ist;

C) eine zum Schallkopf (b) frei wählbare, feste Bestimmungsebene (i), welche durch mindestens drei unterscheidbare, zum Schallkopf (b) fest gewählte Punkte (f;g;h) bestimmt ist, die Positions- und Lagebestimmung des Schallkopfes (b) im Raum zu einer beliebigen Basis (j) durch Längenmessung gewährleistet, und

D) die mindestens drei Punkte der Bestimmungsebene (i) durch Sender (4) belegt sind, dadurch gekennzeichnet, dass

E) die mindestens vier Punkte der Basis (j) durch Empfänger (6) belegt sind.

**2.** Ultraschallaufnahmegerät nach Anspruch 1, da-durch gekennzeichnet, dass die Längenmessun-gen zur Bestimmung der Lage und Position des Schallkopfes (b) mittels elektromagnetischer Wellen durchgeführt werden, wobei Interferenzeffekte ausgenützt und/oder Laufzeitbestimmungen ausgeführt werden.

**3.** Ultraschallaufnahmegerät nach Anspruch 1 oder 2, dadurch gekennzeichnet, dass die Lage- und/oder Positionsbestimmung des Schallkopfes (b) mittels Längenmessung, basierend auf elektromagnetischen Wellen, durchgeführt wird.

**4.** Ultraschallaufnahmegerät nach einem der Ansprüche 1 bis 3, dadurch gekennzeichnet, dass die Bestimmungspunkte durch verschiedene Frequenzen vermessen werden.

**5.** Ultraschallaufnahmegerät nach Anspruch 1 oder 2, welches einen manuell frei bewegbaren Ultraschallkopf (2), ein Ultraschallaufnahmegerät (9), ein Bildverarbeitungssystem (8), und eine den Ultraschallkopf (2), eine Auswerteeinheit (7) und im Raum mindestens zwei elektromagnetischen Wel-len detektierende Sensorsysteme (6) einschlies-sende Positionserfassungseinrichtung (10) um-fasst, und somit die Position und Orientierung des Ultraschallkopfes (2) und damit die Lage und Orientierung der Ultraschallschnittbilder im Raum bestimmt werden kann, dadurch gekennzeichnet, dass am Ultraschallkopf (2) mindestens drei elektromagnetische Wellen abgebende Mittel (4) angebracht sind.

**6.** Ultraschallaufnahmegerät nach Anspruch 5, da-durch gekennzeichnet, dass die elektromagneti-sche Wellen abgebenden Mittel (4) Infrared Light Emitting Dioden (IRED) sind.

**7.** Ultraschallaufnahmegerät nach Anspruch 5, dadurch gekennzeichnet, dass die elektromagnetische Wellen abgebenden Mittel (4) durch eine Lichtquelle gespiesene optische Faserleiter sind.

**8.** Ultraschallaufnahmegerät nach Anspruch 5, da-durch gekennzeichnet, dass die elektromagneti-sche Wellen abgebenden Mittel (4) Fluoreszenzreflektoren sind.

**9.** Ultraschallaufnahmegerät nach Anspruch 5, da-durch gekennzeichnet, dass die elektromagneti-sche Wellen abgebenden Mittel (4) Reflektoren sind.

**10.** Ultraschallaufnahmegerät nach einem der Ansprüche 5 bis 9, dadurch gekennzeichnet, dass die Sen-sorsysteme (6) raumfeste eindimensionale Kame-ras sind und somit durch die Auswerteeinheit (7) die Position und Orientierung des Ultraschallkopfes (2) ermittelt werden kann.

**11.** Ultraschallaufnahmegerät nach einem der Ansprüche 5 bis 9, dadurch gekennzeichnet, dass die Sensorsysteme (6) eindimensionale Kameras sind und nicht raumfest angeordnet sind und die Lage der Kameras durch Aufnahme und Auswertung der Bilder eines raumfesten Passpunktfeldes (12) erfolgt und somit durch die Auswerteeinheit (7) die Position und Orientierung des Ultraschallkopfes (2) ermittelt werden kann.

**12.** Ultraschallaufnahmegerät nach einem der Ansprüche 5 bis 10, dadurch gekennzeichnet, dass die Sensorsysteme (6) raumfest angeordnete eindimensionale Kameras sind und somit die Lage und Orientierung eines nicht raumfesten Passpunktfeldes, das zum Beispiel am Patienten angebracht ist, ermittelt werden kann.

**13.** Ultraschallaufnahmegerät nach einem der Ansprüche 5 bis 9, dadurch gekennzeichnet, dass die mindestens zwei Sensorsysteme (6) raumfeste Kameras (11) sind und somit durch die Auswerteeinheit (7) videogrammetrisch die Position und Orientierung des Ultraschallkopfes (2) ermittelt werden kann.

**14.** Ultraschallaufnahmegerät nach einem der Ansprüche 5 bis 9, dadurch gekennzeichnet, dass die mindestens zwei Sensorsysteme (6) Kameras (11) sind, die nicht raumfest angeordnet sind und die Lage der Kameras (11) durch Aufnahme und Auswertung der Bilder eines raumfesten Passpunktfeldes (12) erfolgt und somit durch die Auswerteeinheit (7) videogrammetrisch die Position und Orientierung des Ultraschallkopfes (2) ermittelt werden kann.

**15.** Ultraschallaufnahmegerät nach einem der Ansprüche 5 bis 9, dadurch gekennzeichnet, dass die mindestens zwei Sensorsysteme (6) raumfeste Kameras (11) sind und somit die Lage und Orientierung eines nicht raumfesten Passpunktfeldes, das zum Beispiel am Patienten angebracht ist, ermittelt werden kann.

16. Ultraschallaufnahmegerät nach einem der Ansprüche 10 bis 15, dadurch gekennzeichnet, dass die Kameras (11) digitale Kameras sind.

17. Ultraschallaufnahmegerät nach einem der Ansprüche 10 bis 15, dadurch gekennzeichnet, dass die Kameras (11) digitale, eindimensionale Kameras sind.

18. Ultraschallaufnahmegerät nach einem der Ansprüche 1 bis 17, dadurch gekennzeichnet, dass der manuell frei bewegbare Ultraschallkopf (2), das Ultraschallaufnahmegerät (9), das Bildverarbeitungssystem (8), und die Positionserfassungseinrichtung (10) mit einem Computer Assisted Surgery System (CAS) verbunden sind.

19. Vorrichtung zur Aufnahme von dreidimensionalen Ultraschallbildern, mit dem Ultraschallaufnahmegerät nach einem der Ansprüche 1 bis 18, welche

A) ein Bildverarbeitungssystem (8), und eine den Ultraschallkopf (2), eine Auswerteeinheit (7) und im Raum mindestens zwei elektromagnetischen Wellen detektierende Sensorsysteme (6) einschliessende Positionserfassungseinrichtung (10) umfasst und somit die Position und Orientierung des Ultraschallkopfes (2) und damit die Lage und Orientierung der Ultraschallschnittbilder im Raum bestimmt werden kann, wobei

B) am Ultraschallkopf (2) mindestens drei elektromagnetische Wellen abgebende Mittel (4) angebracht sind und die Positions- und Lagebestimmung des Ultraschallkopfes (2) im Raum zu einer beliebigen Basis (j) durch Längenmessung gewährleistet wird, dadurch gekennzeichnet, dass

C) die tomographisch erfassten Bilder eines dreidimensionalen Körpers (1) mittels des Bildverarbeitungssystems (8) als dreidimensionaler Datensatz im Computer (15) speicherbar und verarbeitbar sind.

20. Vorrichtung nach Anspruch 19, dadurch gekennzeichnet, dass die Längenmessungen zur Bestimmung der Lage und Position des Ultraschallkopfes (2) mittels elektromagnetischer Wellen durchgeführt werden, wobei Interferenzeffekte ausgenützt und/oder Laufzeitbestimmungen ausgeführt werden.

21. Vorrichtung nach einem der Ansprüche 19 oder 20, dadurch gekennzeichnet, dass die elektromagnetische Wellen abgebenden Mittel (4) optische Leuchtquellen sind.

22. Vorrichtung nach einem der Ansprüche 19 oder 20, dadurch gekennzeichnet, dass die elektromagnetische Wellen abgebenden Mittel (4) Infrared Light Emitting Dioden (IRED) sind.

23. Vorrichtung nach einem der Ansprüche 19 oder 20, dadurch gekennzeichnet, dass die elektromagnetische Wellen abgebenden Mittel (4) durch eine Lichtquelle gespiesene optische Faserleiter sind.

24. Vorrichtung nach einem der Ansprüche 19 oder 20, dadurch gekennzeichnet, dass die elektromagnetische Wellen abgebenden Mittel (4) Fluoreszenzreflektoren sind.

25. Vorrichtung nach einem der Ansprüche 19 oder 20, dadurch gekennzeichnet, dass die elektromagnetische Wellen abgebenden Mittel (4) Reflektoren sind.

26. Vorrichtung nach einem der Ansprüche 19 bis 25, dadurch gekennzeichnet, dass die Sensorsysteme (6) raumfeste eindimensionale Kameras sind und somit durch die Auswerteeinheit (7) die Position und Orientierung des Ultraschallkopfes (2) ermittelt werden kann.

27. Vorrichtung nach einem der Ansprüche 19 bis 25, dadurch gekennzeichnet, dass die Sensorsysteme (6) eindimensionale Kameras sind und nicht raumfest angeordnet sind und die Lage der Kameras durch Aufnahme und Auswertung der Bilder eines raumfesten Passpunktfeldes (12) erfolgt und somit durch die Auswerteeinheit (7) die Position und Orientierung des Ultraschallkopfes (2) ermittelt werden kann.

28. Vorrichtung nach einem der Ansprüche 19 bis 25, dadurch gekennzeichnet, dass die Sensorsysteme (6) raumfest angeordnete eindimensionale Kameras sind und somit die Lage und Orientierung eines nicht raumfesten Passpunktfeldes, das zum Beispiel am Patienten angebracht ist, ermittelt werden kann.

29. Vorrichtung nach einem der Ansprüche 19 bis 25, dadurch gekennzeichnet, dass die mindestens zwei Sensoren (6) raumfeste Kameras (11) sind und somit durch die Auswerteeinheit (7) videogrammetrisch die Position und Orientierung des Ultraschallkopfes (2) ermittelt werden kann.

30. Vorrichtung nach einem der Ansprüche 19 bis 25, dadurch gekennzeichnet, dass die mindestens zwei Sensoren (6) Kameras (11) sind, die nicht raumfest angeordnet sind und die Lage der Kameras (11) durch Aufnahme und Auswertung der Bilder eines raumfesten Passpunktfeldes (12) erfolgt und somit durch die Auswerteeinheit (7) videogrammetrisch die Position und Orientierung des Ultraschallkopfes (2) ermittelt werden kann.

31. Vorrichtung nach einem der Ansprüche 19 bis 25, dadurch gekennzeichnet, dass die mindestens zwei Sensoren (6) raumfeste Kameras (11) sind und somit die Lage und Orientierung eines nicht raumfesten Passpunktfeldes, das zum Beispiel am Patienten angebracht ist, ermittelt werden kann.

32. Vorrichtung nach einem der Ansprüche 19 bis 31, dadurch gekennzeichnet, dass die Kameras (11) digitale Kameras sind.

33. Vorrichtung nach einem der Ansprüche 19 bis 32, dadurch gekennzeichnet, dass der manuell frei bewegbare Ultraschallkopf (2), das Ultraschallaufnahmegerät (9), das Bildverarbeitungssystem (8), und die Positionserfassungseinrichtung (10) mit einem Computer Assisted Surgery System (CAS) verbunden sind.

34. Verfahren zur Aufnahme von dreidimensionalen Ultraschallbildern, welches die Anwendung eines Ultraschallaufnahmegerätes gemäss einem der Ansprüche 1 bis 18, eines Bildverarbeitungssystemes (8), und einer Positionserfassungseinrichtung (10) umfasst, und die Positionserfassungseinrichtung (10) es erlaubt, die Position und Orientierung des Ultraschallkopfes (2) und damit die Lage und Orientierung der Ultraschallschnittbilder im Raum zu bestimmen, wobei
A) die Positionserfassungseinrichtung (10) am Ultraschallkopf (2) angebrachte, elektromagnetische Wellen abgebende Mittel (4), eine Auswerteeinheit (7) und im Raum mindestens zwei diese elektromagnetischen Wellen detektierende Sensorsysteme (6) umfasst und die Positions- und Lagebestimmung des Ultraschallkopfes (2) im Raum zu einer beliebigen Basis (j) durch Längenmessung gewährleistet wird,
dadurch gekennzeichnet, dass
B) die tomographisch erfassten Bilder eines dreidimensionalen Körpers (1) mittels des Bildverarbeitungssystems (8) als dreidimensionaler Datensatz im Computer (15) speicherbar und verarbeitbar sind.

35. Verfahren nach Anspruch 34, dadurch gekennzeichnet, dass die Längenmessungen zur Bestimmung der Lage und Position des Ultraschallkopfes (2) mittels elektromagnetischer Wellen durchgeführt werden, wobei Interferenzeffekte ausgenützt und/oder Laufzeitbestimmungen ausgeführt werden.

36. Verfahren nach einem der Ansprüche 34 oder 35, dadurch gekennzeichnet, dass die elektromagnetische Wellen abgebenden Mittel (4) optische Leuchtquellen sind.

37. Verfahren nach einem der Ansprüche 34 oder 35, dadurch gekennzeichnet, dass die elektromagnetische Wellen abgebenden Mittel (4) Infrared Light Emitting Dioden (IRED) sind.

38. Verfahren nach einem der Ansprüche 34 oder 35, dadurch gekennzeichnet, dass die elektromagnetische Wellen abgebenden Mittel (4) durch eine Lichtquelle gespiesene optische Faserleiter sind.

39. Verfahren nach einem der Ansprüche 34 oder 35, dadurch gekennzeichnet, dass die elektromagnetische Wellen abgebenden Mittel (4) Fluoreszenzreflektoren sind.

40. Verfahren nach einem der Ansprüche 34 oder 35, dadurch gekennzeichnet, dass die elektromagnetische Wellen abgebenden Mittel (4) Reflektoren sind.

41. Verfahren nach einem der Ansprüche 31 bis 40, dadurch gekennzeichnet, dass die Sensorsysteme (6) raumfeste eindimensionale Kameras sind und somit durch die Auswerteeinheit (7) die Position und Orientierung des Ultraschallkopfes (2) ermittelt werden kann.

42. Verfahren nach einem der Ansprüche 34 bis 40, dadurch gekennzeichnet, dass die Sensorsysteme (6) eindimensionale Kameras sind und nicht raumfest angeordnet sind und die Lage der Kameras durch Aufnahme und Auswertung der Bilder eines raumfesten Passpunktfeldes (12) erfolgt und somit durch die Auswerteeinheit (7) die Position und Orientierung des Ultraschallkopfes (2) ermittelt werden kann.

43. Verfahren nach einem der Ansprüche 34 bis 40, dadurch gekennzeichnet, dass die Sensorsysteme (6) raumfest angeordnete eindimensionale Kameras sind und somit die Lage und Orientierung eines nicht raumfesten Passpunktfeldes, das zum Beispiel am Patienten angebracht ist, ermittelt werden kann.

44. Verfahren nach einem der Ansprüche 34 bis 40, dadurch gekennzeichnet, dass die mindestens zwei Sensorsysteme (6) raumfeste Kameras (11) sind und somit durch die Auswerteeinheit (7) videogrammetrisch die Position und Orientierung des Ultraschallkopfes (2) ermittelt werden kann.

45. Verfahren nach einem der Ansprüche 34 bis 40, dadurch gekennzeichnet, dass die mindestens zwei Sensoren (6) Kameras (11) sind, die nicht raumfest angeordnet sind und die Lage der Kameras (11) durch Aufnahme und Auswertung der Bilder eines raumfesten Passpunktfeldes (12) erfolgt und somit durch die Auswerteeinheit (7) videogrammetrisch die Position und Orientierung des Ultraschallkopfes (2) ermittelt werden kann.

46. Verfahren nach einem der Ansprüche 34 bis 40, dadurch gekennzeichnet, dass die mindestens zwei Sensoren (6) raumfeste Kameras (11) sind und somit die Lage und Orientierung eines nicht raumfesten Passpunktfeldes, das zum Beispiel am Patienten angebracht ist, ermittelt werden kann.

47. Verfahren nach einem der Ansprüche 34 bis 46, dadurch gekennzeichnet, dass die Kameras (11) digitale Kameras sind.

48. Verfahren nach einem der Ansprüche 34 bis 47, dadurch gekennzeichnet, dass der manuell frei bewegbare Ultraschallkopf (2), das Ultraschallaufnahmegerät (9), das Bildverarbeitungssystem (8), und die Positionserfassungseinrichtung (10) mit einem Computer Assisted Surgery System (CAS) verbunden sind.

**Claims**

1. Ultrasonographic recording system for the acquisition of ultrasound images by means of electronic data processing, whereby

> A) the position of the tomographic ultrasound images (a) relative to any given spatial base is unambiguously defined;
> B) the position and orientation of the tomographic ultrasound images (a) are determined by the position and orientation of the ultrasound scanning head (b);
> C) a fixed control plane (i) which is freely selectable relative to the ultrasound scanning head (b) and which is defined by at least three distinguishable control points (f;g;h) that are selected in a specific relationship to the ultrasound scanning head (b), ensures the determination of the spatial position and orientation of the ultrasound scanning head (b) relative to any given base (j) by appropriate linear measurement; and
> D) the minimum of three control points of the control plane (i) are taken through transmitters (4),

characterized in that
E) the minimum of four control points of the base (j) are taken through receivers (6).

2. Ultrasonographic recording system as in claim 1, characterized in that the linear measurements serving to determine the position and orientation of the ultrasound scanning head (b) are made using electromagnetic waves under utilization of interference effects and/or run-length measurements.

3. Ultrasonographic recording system as in one of the claims 1 to 2, characterized in that the positional and/or orientational determination of the ultrasound scanning head (b) is obtained by means of linear measurements employing electromagnetic waves.

4. Ultrasonographic recording system as in one of the claims 1 to 3, characterized in that the control points are measured at different frequencies.

5. Ultrasonographic recording system as in claim 1 or 2, incorporating a freely movable, manually guided ultrasound scanning head (2), an ultrasound acquisition device (9), an image processing unit (8) as well as a positional locating device (10) which encompasses the ultrasound scanning head (2), an evaluation unit (7) and at least two intraspatially operating electromagnetic-wave-detecting sensor arrays (6), permitting the determination of the position and orientation of the ultrasound scanning head (2) and thus of the spatial position and orientation of the tomographic ultrasound images, characterized in that the ultrasound scanning head (2) is provided with at least three electromagnetic-wave-emitting devices (4).

6. Ultrasonographic recording system as in claim 5, characterized in that the said electromagnetic-wave-emitting devices (4) are infrared light emitting diodes (IRLEDs).

7. Ultrasonographic recording system as in claim 5, characterized in that the said electromagnetic-wave-emitting devices (4) consist of fiber optics connected to a light source.

8. Ultrasonographic recording system as in claim 5, characterized in that the said electromagnetic-wave-emitting devices (4) are fluorescence reflectors.

9. Ultrasonographic recording system as in claim 5, characterized in that the said electromagnetic-wave-emitting devices (4) are reflectors.

**10.** Ultrasonographic recording system as in one of the claims 5 to 9, characterized in that the sensors (6) are in the form of spatially fixed unidimensional cameras enabling the evaluation unit (7) to determine the position and orientation of the ultrasound scanning head (2).

**11.** Ultrasonographic recording system as in one of the claims 5 to 9, characterized in that the sensors (6) are in the form of unidimensional cameras which are not spatially fixed and that the position of the cameras is determined by the acquisition and evaluation of the images on a spatially fixed control-point reference field (12), enabling the evaluation unit (7) to determine the position and orientation of the ultrasound scanning head (2).

**12.** Ultrasonographic recording system as in one of the claims 5 to 10, characterized in that the sensors (6) are in the form of spatially fixed unidimensional cameras permitting the positional and orientational determination of a spatially variable control-point reference field located for instance on a patient.

**13.** Ultrasonographic recording system as in one of the claims 5 to 9, characterized in that the said minimum of two sensors (6) are in the form of spatially fixed cameras (11) enabling the evaluation unit (7) to videogrammetrically determine the position and orientation of the ultrasound scanning head (2).

**14.** Ultrasonographic recording system as in one of the claims 5 to 9, characterized in that the said minimum of two sensors (6) are in the form of cameras (11) which are not spatially fixed, the position of the cameras (11) being determined by the acquisition and evaluation of the images on a spatially fixed control-point reference field (12), enabling the evaluation unit (7) to videogrammetrically determine the position and orientation of the ultrasound scanning head (2).

**15.** Ultrasonographic recording system as in one of the claims 5 to 9, characterized in that the said minimum of two sensors (6) are in the form of spatially fixed cameras (11) permitting the positional and orientational determination of a spatially variable control-point reference field located for instance on a patient.

**16.** Ultrasonographic recording system as in one of the claims 10 to 15, characterized in that the cameras (11) are digital cameras.

**17.** Ultrasonographic recording system as in one of the claims 10 to 15, characterized in that the cameras (11) are digital, unidimensional cameras.

**18.** Ultrasonographic recording system as in one of the claims 1 to 17, characterized in that the freely movable, manually guided ultrasound scanning head (2), the ultrasound acquisition device (9), the image processing unit (8) and the positional locating device (10) are connected to a computer-assisted surgery system (CAS).

**19.** System for the acquisition of three-dimensional ultrasound images with the ultrasonic recording system according to one of the claims 1 to 18, comprising

A) an image processing unit (8) as well as a positional locating device (10) which encompasses the ultrasound scanning head (2), an evaluation unit (7) and at least two intraspatially operating electromagnetic-wave-detecting sensor arrays (6), permitting the determination of the position and orientation of the ultrasound scanning head (2) and thus of the spatial position and orientation of the tomographic ultrasound images, whereby
B) the ultrasound scanning head (2) is provided with at least three electromagnetic-wave-emitting devices (4), ensuring the spatial determination of the position and orientation of the ultrasound scanning head (2) in relation to any given base (j) through linear measurements,

characterized in that
C) the tomographically obtained images of a three-dimensional body (1) are adapted by means of the image processing unit to be stored and processed as a three-dimensional data set in the computer (15).

**20.** System as in claim 19, characterized in that the linear measurements serving to determine the position and orientation of the ultrasound scanning head (b) are made using electromagnetic waves under utilization of interference effects and/or run-time measurements.

**21.** System as in claim 19 or 20, characterized in that the electromagnetic-wave-emitting devices (4) are optical light sources.

**22.** System as in claim 19 or 20, characterized in that the electromagnetic-wave-emitting devices (4) are infrared light-emitting diodes (IREDs).

**23.** System as in claim 19 or 20, characterized in that the electromagnetic-wave-emitting devices (4) consist of fiber optics connected to a light source.

**24.** System as in claim 19 or 20, characterized in that the electromagnetic-wave-emitting devices (4) are fluorescence reflectors.

25. System as in claim 19 or 20, characterized in that the electromagnetic-wave-emitting devices (4) are reflectors.

26. System as in one of the claims 19 to 25, characterized in that the said sensors (6) are spatially fixed, unidimensional cameras enabling the evaluation unit (7) to determine the position and orientation of the ultrasound scanning head (2).

27. System as in one of the claims 19 to 25, characterized in that the said sensors (6) are unidimensional cameras which are not spatially fixed, the position and orientation of the cameras being determined by the acquisition and evaluation of a spatially fixed control-point reference field (12), enabling the evaluation unit (7) to determine the position and orientation of the ultrasound scanning head (2).

28. System as in one of the claims 19 to 25, characterized in that the said sensors (6) are in the form of spatially fixed, unidimensional cameras permitting the positional and orientational determination of a spatially variable control-point reference field located for instance on a patient.

29. System as in one of the claims 19 to 25, characterized in that the said minimum of two sensors (6) are in the form of spatially fixed cameras (11) enabling the evaluation unit (7) to videogrammetrically determine the position and orientation of the ultrasound scanning head (2).

30. System as in one of the claims 19 to 25, characterized in that the said minimum of two sensors (6) are in the form of cameras (11) which are not spatially fixed, the position of the cameras (11) being determined by the acquisition and evaluation of the images on a spatially fixed control-point reference field (12), enabling the evaluation unit (7) to videogrammetrically determine the position and orientation of the ultrasound scanning head (2).

31. System as in one of the claims 19 to 25, characterized in that the said minimum of two sensors (6) are in the form of spatially fixed cameras (11) permitting the positional and orientational determination of a spatially variable control-point reference field located for instance on a patient.

32. System as in one of the claims 19 to 31, characterized in that the cameras (11) are digital cameras.

33. System as in one of the claims 19 to 32, characterized in that the freely movable, manually guided ultrasound scanning head (2), the ultrasound acquisition device (9), the image processing unit (8) and the positional locating device (10) are connected to a computer-assisted surgery system (CAS).

34. Procedure for the acquisition of three-dimensional ultrasound images including the application of a ultrasonographic recording system according to one of the claims 1 to 18, an image processing unit (8) and a positional locating device (10), and the positional locating device (10) permits the positional and orientational determination of the ultrasound scanning head (2) and thus of the spatial determination of the position and orientation of the tomographic images, whereby
A) the positional locating device (10) encompasses electromagnetic-wave-emitting devices (4) mounted on the ultrasound scanning head (2), an evaluation unit (7) and at least two intraspatially operating sensor arrays (6) detecting the said electromagnetic waves, ensuring the determination of the position and orientation of the ultrasound scanning head (2) in relation to any given base (j) through linear measurements,
characterized in that
B) the tomographically obtained images of a three-dimensional body (1) are adapted by means of the image processing unit to be stored and processed as a three-dimensional data set in the computer (15).

35. Procedure as in claim 34, characterized in that the linear measurements serving to determine the position and orientation of the ultrasound scanning head (b) are made using electromagnetic waves under utilization of interference effects and/or run-length measurements.

36. Procedure as in claim 34 or 35, characterized in that the electromagnetic-wave-emitting devices (4) are optical light sources.

37. Procedure as in claim 34 or 35, characterized in that the electromagnetic-wave-emitting devices (4) are infrared light-emitting diodes (IRLEDs).

38. Procedure as in claim 34 or 35, characterized in that the electromagnetic-wave-emitting devices (4) consist of fiber optics connected to a light source.

39. Procedure as in claim 34 or 35, characterized in that the electromagnetic-wave-emitting devices (4) are fluorescence reflectors.

40. Procedure as in claim 34 or 35, characterized in that the electromagnetic-wave-emitting devices (4) are reflectors.

41. Procedure as in one of the claims 34 to 40, characterized in that the sensors (6) are spatially fixed unidimensional cameras, enabling the evaluation unit (7) to determine the position and orientation of the ultrasound scanning head (2).

**42.** Procedure as in one of the claims 34 to 40, characterized in that the sensors (6) are in the form of unidimensional cameras which are not spatially fixed, the position of the cameras being determined by the acquisition and evaluation of the images on a spatially fixed control-point reference field (12), enabling the evaluation unit (7) to determine the position and orientation of the ultrasound scanning head (2).

**43.** Procedure as in one of the claims 34 to 40, characterized in that the sensors (6) are in the form of spatially fixed, unidimensional cameras permitting the positional and orientational determination of a spatially variable control-point reference field located for instance on a patient.

**44.** Procedure as in one of the claims 34 to 40, characterized in that the said minimum of two sensors (6) are in the form of spatially fixed cameras (11) enabling the evaluation unit (7) to videogrammetrically determine the position and orientation of the ultrasound scanning head (2).

**45.** Procedure as in one of the claims 34 to 40, characterized in that the said minimum of two sensors (6) are in the form of cameras (11) which are not spatially fixed, the position of the cameras (11) being determined by the acquisition and evaluation of the images on a spatially fixed control-point reference field (12), enabling the evaluation unit (7) to videogrammetrically determine the position and orientation of the ultrasound scanning head (2).

**46.** Procedure as in one of the claims 34 to 40, characterized in that the said minimum of two sensors (6) are in the form of spatially fixed cameras (11) permitting the positional and orientational determination of a spatially variable control-point reference field located for instance on a patient.

**47.** Procedure as in one of the claims 34 to 46, characterized in that the cameras (11) are digital cameras.

**48.** Procedure as in one of the claims 34 to 47, characterized in that the freely movable, manually guided ultrasound scanning head (2), the ultrasound acquisition device (9), the image processing unit (8) and the positional locating device (10) are connected to a computer-assisted surgery system (CAS).

## Revendications

**1.** Appareil d'enregistrement par ultrasons pour l'enregistrement d'images obtenues par ultrasons au moyen d'un traitement électronique de données, dans lequel

A) la position par rapport à une base quelconque dans l'espace des images tomographiques (a) obtenues par ultrasons est déterminée de manière univoque ;
B) la position des images tomographiques (a) obtenues par ultrasons est déterminée par la position de la tête acoustique (b) ;
C) un plan de référence fixe (i), qui peut être sélectionné librement par rapport à la tête acoustique (b) et qui est déterminé par au moins trois points (f ; g ; h) sélectionnés librement par rapport à la tête acoustique (b), assure la détermination de la position de la tête acoustique (b) dans l'espace par rapport à une base (j) quelconque, par mesure de longueur, et
D) les trois points au moins présents du plan de détermination (i) sont occupés par des émetteurs (4),

caractérisé en ce que
E) les quatre points au moins présents de la base (j) sont occupés par des récepteurs (6).

**2.** Appareil d'enregistrement par ultrasons selon la revendication 1, caractérisé en ce que les mesures de longueur en vue de la détermination de la position de la tête acoustique (b) sont exécutées au moyen d'ondes électromagnétiques, et en ce que l'on utilise des effets d'interférence et/ou que l'on exécute des déterminations de temps de parcours.

**3.** Appareil d'enregistrement par ultrasons selon la revendication 1 ou 2, caractérisé en ce que la détermination de la position de la tête acoustique (b) est réalisée au moyen d'une mesure de longueur basée sur des ondes électromagnétiques.

**4.** Appareil d'enregistrement par ultrasons selon l'une des revendications 1 à 3, caractérisé en ce que les points de détermination sont mesurés à différentes fréquences.

**5.** Appareil d'enregistrement par ultrasons selon la revendication 1 ou 2, qui comporte une tête à ultrasons (2) apte à être déplacée librement à la main, un appareil d'enregistrement par ultrasons (9), un système de traitement d'image (8) et un dispositif de détermination de position (10) comportant la tête à ultrasons (2), une unité d'évaluation (7) et un système de détecteurs (6) détectant au moins deux ondes électromagnétiques dans l'espace, et ainsi la position et l'orientation de la tête à ultrasons (2) et donc la position et l'orientation dans l'espace des images tomographiques obtenues par ultrasons peuvent être déterminées, caractérisé en ce que au moins trois moyens (4) émettant des ondes électromagnétiques sont installés sur la tête à ultrasons (2).

6. Appareil d'enregistrement par ultrasons selon la revendication 5, caractérisé en ce que les moyens (4) émettant des ondes électromagnétiques sont des diodes luminescentes dans l'infrarouge ("Infrared Light Emetting Diodes" - IRED).

7. Appareil d'enregistrement par ultrasons selon la revendication 5, caractérisé en ce que les moyens (4) émettant des ondes électromagnétiques sont des conducteurs optiques à fibres alimentés par une source de lumière.

8. Appareil d'enregistrement par ultrasons selon la revendication 5, caractérisé en ce que les moyens (4) émettant des ondes électromagnétiques sont des réflecteurs à fluorescence.

9. Appareil d'enregistrement par ultrasons selon la revendication 5, caractérisé en ce que les moyens (4) émettant des ondes électromagnétiques sont des réflecteurs.

10. Appareil d'enregistrement par ultrasons selon l'une des revendications 5 à 9, caractérisé en ce que les systèmes de capteurs (6) sont des caméras unidimensionnelles fixes dans l'espace qui permettent à l'unité d'évaluation (7) de déterminer la position et l'orientation de la tête à ultrasons (2).

11. Appareil d'enregistrement par ultrasons selon l'une des revendications 5 à 9, caractérisé en ce que les systèmes de capteurs (6) sont des caméras unidimensionnelles et ne sont pas disposés fixes dans l'espace, et la position des caméras s'effectue par enregistrement et évaluation des images d'un champ de points d'adaptation (12) fixes dans l'espace, ce qui permet à l'unité d'évaluation (7) de déterminer la position et l'orientation de la tête à ultrasons (2).

12. Appareil d'enregistrement par ultrasons selon l'une des revendications 5 à 10, caractérisé en ce que les systèmes de capteurs (6) sont des caméras unidimensionnelles disposées fixes dans l'espace qui permettent de calculer la position et l'orientation d'un champ de points d'adaptation non fixes dans l'espace et par exemple placé sur le patient.

13. Appareil d'enregistrement par ultrasons selon l'une des revendications 5 à 9, caractérisé en ce que les deux systèmes de capteurs (6) au moins présents sont des caméras (11) fixes dans l'espace, ce qui permet à l'unité d'évaluation (7) de déterminer la position et l'orientation de la tête à ultrasons (2) sous la forme d'un vidéogramme.

14. Appareil d'enregistrement par ultrasons selon l'une des revendications 5 à 9, caractérisé en ce que les deux systèmes de capteurs (6) au moins présents sont des caméras (11) qui ne sont pas disposées fixes dans l'espace, et la position des caméras (11) s'effectue par enregistrement et évaluation des images d'un champ de points d'adaptation (12) fixes dans l'espace, ce qui permet à l'unité d'évaluation (7) de déterminer la position et l'orientation de la tête à ultrasons (2) sous la forme d'un vidéogramme.

15. Appareil d'enregistrement par ultrasons selon l'une des revendications 5 à 9, caractérisé en ce que les deux systèmes de capteurs (6) au moins présents sont des caméras (11) fixes dans l'espace qui permettent de déterminer la position et l'orientation d'un champ de points d'adaptation non fixes dans l'espace et par exemple placés sur le patient.

16. Appareil d'enregistrement par ultrasons selon l'une des revendications 10 à 15, caractérisé en ce que les caméras (11) sont des caméras numériques.

17. Appareil d'enregistrement par ultrasons selon l'une des revendications 10 à 15, caractérisé en ce que les caméras (11) sont des caméras numériques unidimensionnelles.

18. Appareil d'enregistrement par ultrasons selon les revendications 1 à 17, caractérisé en ce que la tête à ultrasons (2) apte à être déplacée librement à la main, l'appareil d'enregistrement par ultrasons (9), le système de traitement d'image (8) et le dispositif (10) de détection de position sont reliés à un système de chirurgie assistée par ordinateur ("Computer Assisted Surgery" - CAS).

19. Dispositif d'enregistrement d'images tridimensionnelles obtenues par ultrasons, au moyen de l'appareil d'enregistrement par ultrasons selon l'une des revendications 1 à 18, lequel dispositif comporte

　　A) un système de traitement d'image (8) et un dispositif (10) de détection de position qui comprend la tête à ultrasons (2), une unité d'évaluation (7) et au moins deux systèmes de capteurs (6) détectant des ondes électromagnétiques dans l'espace, ce qui permet de déterminer la position et l'orientation de la tête à ultrasons (2) et donc la position et l'orientation dans l'espace des images tomographiques obtenues par ultrasons peuvent, tandis que

B) sur la tête à ultrasons sont installés au moins trois moyens (4) émettant des ondes électromagnétiques, et la détermination de la position de la tête à ultrasons (2) dans l'espace par rapport à une base quelconque (j) est assurée par mesure de longueur,

caractérisé en ce que

C) les images obtenues par tomographie d'un corps tridimensionnel (1) peuvent être conservées en mémoire et traitées sous forme de jeu de données tridimensionnelles dans l'ordinateur (15) du système de traitement d'image (8).

20. Dispositif selon la revendication 19, caractérisé en ce que les mesures de longueur en vue de la détermination de la position de la tête à ultrasons (2) sont réalisées au moyen d'ondes électromagnétiques, et l'on utilise des effets d'interférence et/ou l'on réalise des déterminations de temps de parcours.

21. Dispositif selon l'une des revendications 19 ou 20, caractérisé en ce que les moyens (4) émettant des ondes électromagnétiques sont des sources optiques de lumière.

22. Dispositif selon l'une des revendications 19 ou 20, caractérisé en ce que les moyens (4) émettant des ondes électromagnétiques sont des diodes luminescentes dans l'infrarouge (IRED).

23. Dispositif selon l'une des revendications 19 ou 20, caractérisé en ce que les moyens (4) émettant des ondes électromagnétiques sont des conducteurs optiques à fibres alimentés par une source de lumière.

24. Dispositif selon l'une des revendications 19 ou 20, caractérisé en ce que moyens (4) émettant des ondes électromagnétiques sont des réflecteurs à fluorescence.

25. Dispositif selon l'une des revendications 19 ou 20, caractérisé en ce que moyens (4) émettant des ondes électromagnétiques sont des réflecteurs.

26. Dispositif selon l'une des revendications 19 à 25, caractérisé en ce que les systèmes de capteurs (6) sont des caméras unidimensionnelles fixes dans l'espace qui permettent à l'unité d'évaluation (7) de déterminer la position et l'orientation de la tête à ultrasons (2).

27. Dispositif selon l'une des revendications 19 à 25, caractérisé en ce que les systèmes de capteurs (6) sont des caméras unidimensionnelles et sont disposés non fixes dans l'espace, et la localisation des caméras s'effectue par enregistrement et évaluation des images d'un champ de points d'adaptation (12) fixes dans l'espace, ce qui permet à l'unité d'évaluation (7) de déterminer la position et l'orientation de la tête à ultrasons (2).

28. Dispositif selon l'une des revendications 19 à 25, caractérisé en ce que les systèmes de capteurs (6) sont des caméras unidimensionnelles disposées fixes dans l'espace qui permettent de déterminer la position et l'orientation d'un champ de points d'adaptation non fixes dans l'espace et par exemple placés sur le patient.

29. Dispositif selon l'une des revendications 19 à 25, caractérisé en ce que les deux capteurs (6) au moins présents sont des caméras (11) fixes dans l'espace qui permettent à l'unité d'évaluation (7) de déterminer la position et l'orientation de la tête à ultrasons (2) sous la forme d'un vidéogramme.

30. Dispositif d'enregistrement par ultrasons selon l'une des revendications 19 à 25, caractérisé en ce que les deux systèmes de capteurs (6) au moins présents sont des caméras (11) qui ne sont pas disposées fixes dans l'espace, et la localisation des caméras (11) s'effectue par enregistrement et évaluation des images d'un champ de points d'adaptation (12) fixes dans l'espace, ce qui permet à l'unité d'évaluation (7) de déterminer la position et l'orientation de la tête à ultrasons (2) sous la forme d'un vidéogramme.

31. Dispositif d'enregistrement par ultrasons selon l'une des revendications 19 à 25, caractérisé en ce que les deux systèmes de capteurs (6) au moins présents sont des caméras (11) fixes dans l'espace qui permettent de déterminer la position et l'orientation d'un champ de points d'adaptation non fixes dans l'espace et par exemple placés sur le patient.

32. Dispositif d'enregistrement par ultrasons selon l'une des revendications 19 à 31, caractérisé en ce que les caméras (11) sont des caméras numériques.

33. Dispositif d'enregistrement par ultrasons selon les revendications 19 à 32, caractérisé en ce que la tête à ultrasons (2) apte à être déplacée librement à la main, l'appareil d'enregistrement par ultrasons (9), le système de traitement d'image (8) et le dispositif (10) de détection de position sont reliés à un système de chirurgie assistée par ordinateur ("Computer Assisted Surgery" - CAS).

34. Procédé d'enregistrement d'images tridimensionnelles obtenues par ultrasons, lequel procédé comporte l'utilisation d'un appareil d'enregistrement par ultrasons selon l'une des revendications 1 à 18, d'un système de traitement d'image (8) et d'un dispositif (10) de détection de position, le dispositif (10) de détection de position permettant de déterminer la position et l'orientation de la tête à ultrasons (2) et donc la position et l'orientation dans l'espace des images tomographiques obtenues par ultrasons, tandis que

A) le dispositif (10) de détection de position comprend des moyens (4) installés sur la tête à ultrasons (2) et émettant des ondes électromagnétiques, une unité d'évaluation (7) et au moins deux systèmes de capteurs (6) détectant ces ondes électromagnétiques dans l'espace, la détermination de position de la tête à ultrasons (2) dans l'espace par rapport à une base (j) quelconque étant assurée par mesure de longueur,

caractérisé en ce que

B) les images obtenues par tomographie d'un corps tridimensionnel (1) au moyen du système de traitement d'image (8) peuvent être mises en mémoire et traitées sous forme de jeu de données tridimensionnelles dans l'ordinateur (15).

35. Procédé selon la revendication 34, caractérisé en ce que les mesures de longueur en vue de la détermination de la position de la tête à ultrasons (2) sont réalisées au moyen d'ondes électromagnétiques, et l'on utilise des effets d'interférence et/ou l'on réalise des déterminations de temps de parcours.

36. Procédé selon l'une des revendications 34 ou 35, caractérisé en ce que les moyens (4) émettant des ondes électromagnétiques sont des sources optiques de lumière.

37. Procédé selon l'une des revendications 34 ou 35, caractérisé en ce que les moyens (4) émettant des ondes électromagnétiques sont des diodes luminescentes dans l'infrarouge (IRED).

38. Procédé selon l'une des revendications 34 ou 35, caractérisé en ce que les moyens (4) émettant des ondes électromagnétiques sont des conducteurs optiques à fibres alimentés par une source de lumière.

39. Procédé selon l'une des revendications 34 ou 35, caractérisé en ce que moyens (4) émettant des ondes électromagnétiques sont des réflecteurs à fluorescence.

40. Procédé selon l'une des revendications 34 ou 35, caractérisé en ce que moyens (4) émettant des ondes électromagnétiques sont des réflecteurs.

41. Procédé selon l'une des revendications 31 à 40, caractérisé en ce que les systèmes de capteurs (6) sont des caméras unidimensionnelles fixes dans l'espace qui permettent à l'unité d'évaluation (7) de déterminer la position et l'orientation de la tête à ultrasons (2).

42. Procédé selon l'une des revendications 34 à 40, caractérisé en ce que les systèmes de capteurs (6) sont des caméras unidimensionnelles et sont disposés non fixes dans l'espace, et la position des caméras s'effectue par enregistrement et évaluation des images d'un champ de points d'adaptation (12) fixes dans l'espace, ce qui permet à l'unité d'évaluation (7) de déterminer la position et l'orientation de la tête à ultrasons (2).

43. Procédé selon l'une des revendications 34 à 40, caractérisé en ce que les systèmes de capteurs (6) sont des caméras unidimensionnelles disposées fixes dans l'espace qui permettent de déterminer la position et l'orientation d'un champ de points d'adaptation non fixes dans l'espace et par exemple placés sur le patient.

44. Procédé selon l'une des revendications 34 à 40, caractérisé en ce que les deux capteurs (6) au moins présents sont des caméras (11) fixes dans l'espace qui permettent à l'unité d'évaluation (7) de déterminer la position et l'orientation de la tête à ultrasons (2) sous la forme d'un vidéogramme.

45. Procédé d'enregistrement par ultrasons selon l'une des revendications 34 à 40, caractérisé en ce que les deux systèmes de capteurs (6) au moins présents sont des caméras (11) qui ne sont pas disposées fixes dans l'espace, et la position des caméras (11) s'effectue par enregistrement et évaluation des images d'un champ de points d'adaptation (12) fixes dans l'espace, ce qui permet à l'unité d'évaluation (7) de déterminer la position et l'orientation de la tête à ultrasons (2) sous la forme d'un vidéogramme.

46. Procédé d'enregistrement par ultrasons selon l'une des revendications 34 à 40, caractérisé en ce que les deux systèmes de capteurs (6) au moins présents sont des caméras (11) fixes dans l'espace qui permettent de déterminer la position et l'orientation d'un champ de points d'adaptation non fixes dans l'espace et par exemple placés sur le patient.

47. Procédé d'enregistrement par ultrasons selon l'une des revendications 34 à 46, caractérisé en ce que les caméras (11) sont des caméras numériques.

48. Procédé d'enregistrement par ultrasons selon les revendications 34 à 47, caractérisé en ce que la tête à ultrasons (2) apte à être déplacée librement à la main, l'appareil d'enregistrement par ultrasons (9), le système de traitement d'image (8) et le dispositif (10) de détection de position sont reliés à un système de chirurgie assistée par ordinateur ("Computer Assisted Surgery" - CAS).

Fig. 1

Fig. 2

Fig.3

Fig. 4